(19) Europäisches Patentamt
European Patent Office
Office européen des brevets

(11) EP 3 515 564 B1

# (12) EUROPEAN PATENT SPECIFICATION

(45) Date of publication and mention of the grant of the patent:
**10.05.2023 Bulletin 2023/19**

(21) Application number: **17772274.1**

(22) Date of filing: **15.09.2017**

(51) International Patent Classification (IPC):
***A61Q 19/08*** (2006.01) ***A61K 8/04*** (2006.01)
***A61K 8/02*** (2006.01) ***A61Q 19/00*** (2006.01)
***A61K 8/34*** (2006.01) ***A61K 8/37*** (2006.01)
***A61K 8/31*** (2006.01) ***A61K 8/41*** (2006.01)
***A61K 8/36*** (2006.01) ***A61K 8/42*** (2006.01)
***A61K 8/44*** (2006.01) ***A61K 8/60*** (2006.01)
***A61K 8/64*** (2006.01) ***A61K 8/67*** (2006.01)
***A61K 8/86*** (2006.01)

(52) Cooperative Patent Classification (CPC):
**A61Q 19/08; A61K 8/0295; A61K 8/046; A61K 8/315; A61K 8/342; A61K 8/345; A61K 8/361; A61K 8/375; A61K 8/416; A61K 8/42; A61K 8/44; A61K 8/604; A61K 8/64; A61K 8/671; A61K 8/675;** (Cont.)

(86) International application number:
**PCT/US2017/051688**

(87) International publication number:
**WO 2018/053213 (22.03.2018 Gazette 2018/12)**

## (54) FOAM COMPOSITIONS, AEROSOL PRODUCTS, AND METHODS OF USING THE SAME TO IMPROVE SENSORY BENEFITS TO THE SKIN

SCHAUMZUSAMMENSETZUNGEN, AEROSOLPRODUKTE UND VERFAHREN ZUR VERWENDUNG DAVON ZUR VERBESSERUNG DES SENSORISCHEN NUTZENS FÜR DIE HAUT

COMPOSITION DE MOUSSE, PRODUITS AÉROSOLS ET LEURS PROCÉDÉ D'UTILISATION POUR AMÉLIORER LES AVANTAGES SENSORIELS SUR LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.09.2016 US 201615268905**
**19.09.2016 US 201615268922**
**26.09.2016 US 201615275908**
**26.09.2016 US 201615275945**

(43) Date of publication of application:
**31.07.2019 Bulletin 2019/31**

(73) Proprietor: **The Procter & Gamble Company**
**Cincinnati, OH 45202 (US)**

(72) Inventors:
- **JANSEN, Joseph, Harry**
  **Cincinnati, Ohio 45202 (US)**
- **ZUKOWSKI, Joseph, Michael**
  **Singapore 138547 (SG)**
- **SUNKEL, Jorge, Max**
  **Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-00/71093**
**WO-A1-2016/153946**
**WO-A2-2008/038140**
**WO-A2-2009/087578**
**WO-A2-2013/057066**
**US-A1- 2004 191 205**
**US-A1- 2005 205 086**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE GNPD [Online] MINTEL; 1 June 2015 (2015-06-01), Norma Sensomed: "Panthenol Foam Spray", XP002775993, Database accession no. 3244591**
- **DATABASE GNPD [Online] MINTEL; 1 July 2008 (2008-07-01), Novartis Consumer Health: "Aftersun Foam Spray", XP002775994, Database accession no. 939546**



(52) Cooperative Patent Classification (CPC): (Cont.)
**A61K 8/86; A61Q 19/00;** A61K 2800/30

## Description

TECHNICAL FIELD

The present disclosure generally relates to foam compositions, aerosol products, and methods of using the same to improve sensory benefits to the skin of a consumer.

BACKGROUND

There are many types of skin care products that are commercially available or otherwise known in the art, and there are many factors that can contribute to the purchase intent of a consumer when looking for such products. Critical among these factors are delivery of skin care actives and the sensory benefits that the skin care product can provide. Skin care actives include a variety of compounds that improve the appearance of skin, acutely and chronically. As you increase the amount of active in a skin care product, you increase the benefits to the skin. But as you increase active delivery, you often decrease the sensory experience to the consumer. As such, there is a consistent desire to develop new ways to deliver larger amounts of skin care actives and improving the positive sensory experience to consumers.

Skin care products have often employed polymers, often thickeners, as a way to manage rheological properties to promote performance benefits. However, some of these polymers are not optimized to provide the desired sensory benefits. For example, elevated polymer concentrations, relative to evaporating fluids, can thicken fluids that remain on the skin during product application and subsequent dry-down, resulting in tack, drag, stickiness, or other negative sensory aspects. Further, such negative aspects can continue after the dry-down phase as a result of sweating and humidity fluctuations.

Using a foam composition is one way to reduce or eliminate the use of polymers. For example, foams can use air to thicken a product in place of polymers. Thus, foams can convey a desired rich and creamy aesthetic while reducing or eliminating the negative sensory aspects associated with the use of polymers. Further, foams can easily absorb into the skin as they can rapidly break down into fluids. However, certain foam compositions can lack the stability that may otherwise be provided by skin care products with polymers. Application of foams lacking the necessary stability can also result in a negative sensory experience for a consumer.

Therefore, what is desired is a skin care product in the form of a foam composition, which possesses robust stability and an ability to provide desired sensory benefits.

A foamable composition has been described in US 2004 191205, example 5.

SUMMARY

In accordance with one example of the present invention there is provided a liquid foamable composition comprising from about 0.5% to about 7%, by weight, of surfactants selected from the group consisting of cationic surfactants, nonionic surfactants, and mixtures thereof. Further there is from about 1% to about 15%, by weight, of a fatty alcohol, and from about 12% to about 25%, by weight, of a skin care active selected from the group consisting of niacinamide, hexyldecanol, a peptide, panthenol, undeylenoyl phenylalanine, retinyl propionate and mixtures thereof, wherein the composition comprises 5% to 30% by weight of a skin care active.

Water is also present, preferably at a concentration of from about 20% to about 85%, by weight.

The surfactant and the fatty alcohol combine to form liquid crystal structures, wherein the liquid crystal structures are of a phase selected from the group consisting of bicontinuous cubic, hexagonal, inverse cubic, lamellar gel, micellar cubic, reverse hexagonal columnar, and combinations thereof. Preferably the liquid crystal structures are of a lamellar gel phase.

In yet another aspect of this invention there is provided from about 5% to about 50%, by weight, of a polyol humectant, which preferably contains at least 50% glycerin, and more preferably the polyol humectant contains at least 75% glycerin. The nonionic surfactant, when present, is preferably selected from the group consisting of Cetearyl Glucoside, Cetearyl Alcohol, Stearic Acid, PEG-100 Stearate, glyceryl monostearate and mixtures thereof. The cationic surfactant, when present, preferably is selected from the group consisting of a quaternary ammonium compound, preferably the quaternary ammonium compound comprises one or more of behenyl trimethyl ammonium chloride, stearamidopropyl dimethylamine, behentrimonium methosulfate, behenylamidopropyl dimethylamine, stearyl ethylhexyldimonium methosulfate, dicetyldimonium chloride, and ditallow dimethyl ammonium chloride. The fatty alcohol preferably has a melting point of about 25°C or higher, and can be selected from the group consisting of behenyl alcohol, cetyl alcohol, batyl alcohol, stearyl alcohol, and mixtures thereof.

The present invention preferably comprises a propellant that is selected from the group consisting of hydrocarbons (A46, A70), hydrofluoro-olefin, carbon dioxide, and mixtures thereof. The liquid foamable composition is preferably substantially free of a thickening agent, and preferably the foam has a specific gravity of less than 0.6.

The foamable compositions of the present invention allow for up to three times the delivered concentration of skin care active, without a decrease in sensory appeal. The present invention allows for the removal of polymeric thickeners and rheological modifiers, again, without degrading the consumer sensory experience.

BRIEF DESCRIPTION OF THE DRAWINGS

FIG. 1 depicts a side elevational view in partial section of an assembled valve mounted to a container according to one example.
FIG. 2 depicts a schematic cross-sectional view of an inner bag housed within a container according to another example.
FIG. 3 depicts a front view of the inner bag of FIG. 2.

DETAILED DESCRIPTION

I. Definitions

As used herein, the following terms shall have the meaning specified thereafter:

"Non-volatile," as it relates to at least fatty alcohols and silicones, can refer to having a boiling point at 1.0 atmospheres of about 260°C or greater, about 275°C or greater, or about 300°C or greater.
"Polymer" can refer to materials formed by polymerization of one type of monomer or formed by polymerization of two or more types of monomers (i.e., copolymers).
"Water soluble" can refer to being sufficiently soluble in water to form a solution that is substantially clear to a naked eye at a concentration of 0.1% in water (distilled or equivalent) at 25°C. The polymer can be sufficiently soluble to form a substantially clear solution at 0.5% concentration in water, and likely to form a substantially clear solution at 1.0% concentration in water.

II. Foam Compositions

Surprisingly, it was found that, due to the use of a surfactant and fatty acid blend in a foamable composition, a very rich and creamy foam composition with robust stability results which provides desired high levels of skin care actives and a high level of sensory benefits can be achieved. The foam composition can be formed from the combination of a liquid foamable composition with a propellant. A liquid composition is generally foamable if it has the ability to entrain or entrap gas (e.g., carbon dioxide). The liquid foamable composition, can be based on a combination of a skin care active, a cationic surfactant (e.g., typically a quaternary ammonium compound), a non-ionic surfactant, and a fatty alcohol.
Essential ingredients, as well as a non-exclusive list of optional ingredients, are described below.

A. Liquid Foamable Composition

A liquid foamable composition can include cationic surfactant (e.g., typically a quaternary ammonium compound), a non-ionic surfactant, a skin care active, a fatty alcohol, water, and other optional ingredients (e.g., glycerin, a super-absorbent polymer). Specific types and ranges for these components are described herein.

Cationic Surfactants

Cationic surfactants suitable for use in the liquid foamable composition can include amino or quaternary ammonium moieties. Additional suitable cationic surfactants are disclosed in the following documents:
M.C. Publishing Co., McCutcheon's, Detergents & Emulsifiers, (North American edition 1979); Schwartz, et al., Surface Active Agents, Their Chemistry and Technology, New York, Interscience Publishers, 1949; U.S. Patent No. 3,155,591, Hilfer, issued Nov. 3, 1964; U.S. Patent No. 3,929,678, Laughlin et al., issued Dec. 30, 1975; U.S. Patent No. 3,959,461, Bailey et al., issued May 25, 1976; and U.S. Patent No. 4,387,090, Bolich, Jr., issued Jun. 7, 1983.
Suitable quaternary ammonium compounds can include those of the general formula:
$[NR1,R2,R3,R4]^{+}\cdot X^{-}$
wherein R1 to R4 can independently be an aliphatic group of from about 1 to about 22 carbon atoms or an aromatic, alkoxy, polyoxyalkylene, alkylamido, hydroxyalkyl, aryl, or alkylaryl group having from about 1 to about 22 carbon atoms; and $X^{-}$ can be a salt-forming anion, such as those selected from halogen (e.g., chloride, bromide, iodide), acetate, citrate,

lactate, glycolate, phosphate nitrate, sulfate, and alkylsulfate radicals.

Such aliphatic groups can contain, in addition to carbon and hydrogen atoms, either linkages or other groups, such as amino groups. The longer-chain aliphatic groups (e.g., those of about 12 carbons, or higher) can be saturated or unsaturated. Mono-long alkyl quaternized ammonium salt cationic surfactants can include behenyl trimethyl ammonium salt, stearyl trimethyl ammonium salt, cetyl trimethyl ammonium salt, and hydrogenated tallow alkyl trimethyl ammonium salt. Di-long chain (e.g., di $C_{12}$-$C_{22}$, $C_{16}$-$C_{18}$, aliphatic, alkyl) and di-short chain (e.g., $C_1$-$C_3$ alkyl, $C_1$-$C_2$ alkyl) ammonium salts can also be employed. Other suitable quaternary ammonium salt useful as cationic surfactants are described in U.S. Patent No. 8,936,798.

Salts of primary, secondary, and tertiary fatty amines can also be suitable cationic surfactant materials. The alkyl groups of such amines can have from about 12 to about 22 carbon atoms, and may be substituted or unsubstituted. Such amines can include stearamidopropyl dimethylamine, behenylamidopropyl dimethylamine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E.O.) stearylamine, dihydroxy ethyl stearylamine, and arachidylbehenylamine. Suitable amine salts can include halogen, acetate, phosphate, nitrate, citrate, lactate, and alkyl sulfate salts. Such salts can include stearylamine hydrochloride, soyamine chloride, stearylamine formate, N-tallowpropane diamine dichloride, and stearamidopropyl dimethylamine citrate. Suitable cationic amine surfactants for the liquid foamable composition are disclosed in U.S. Patent No. 4,275,055, Nachtigal, et al., issued Jun. 23, 1981.

In certain examples, suitable cationic surfactants can include behenyl trimethyl ammonium chloride, stearyl ethylhexyldimonium methosulfate, dicetyldimonium chloride, ditallow dimethyl ammonium chloride, GENAMIN® CTAC (i.e., cetyl trimethyl ammonium chloride), esterquats (e.g., tetradecyl betainester chloride), diesterquats (e.g., dipalmitylethyl dimethyl ammonium chloride, ARMOCARE® VGH70 of Akzo, Germany), or a mixture of distearoylethyl hydroxyethylmonium methosulfate and Cetearyl Alkohol (DEHYQUART® F-75 of Henkel, Germany).

In certain examples, cationic surfactants (e.g., quaternary ammonium compounds) can be included at concentration levels from about 0.05% to about 5%, by weight, of the liquid foamable composition, and in certain examples, from about 1% to about 4%, by weight of the liquid foamable composition. Quaternary ammonium compounds may comprise one or more of behenyl trimethyl ammonium chloride, stearamidopropyl dimethylamine, behentrimonium methosulfate ("BTMS"), behenylamidopropyl dimethylamine, stearyl ethylhexyldimonium methosulfate, dicetyldimonium chloride, and ditallow dimethyl ammonium chloride.

Nonionic Surfactants

Surfactants useful in the present invention may also be selected from nonionic surfactants. Among the nonionic surfactants that are useful herein are those that can be broadly defined as condensation products of long chain alcohols, e.g. C8-30 alcohols, with sugar or starch polymers, i.e., glycosides. These compounds can be represented by the formula $(S)_n$-O-R wherein S is a sugar moiety such as glucose, fructose, mannose, and galactose; n is an integer of from about 1 to about 1000, and R is a C8-30 alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples of these surfactants include those wherein S is a glucose moiety, R is a C8-20 alkyl group, and n is an integer of from about 1 to about 9. Commercially available examples of these surfactants include decyl polyglucoside (available as APG 325 CS from Henkel) and lauryl polyglucoside (available as APG 600 CS and 625 CS from Henkel).

Other useful nonionic surfactants include the condensation products of alkylene oxides with fatty acids (i.e. alkylene oxide esters of fatty acids). These materials have the general formula $RCO(X)_nOH$ wherein R is a C10-30 alkyl group, X is $-OCH_2CH_2-$ (i.e. derived from ethylene glycol or oxide) or $-OCH_2CHCH_3-$ (i.e. derived from propylene glycol or oxide), and n is an integer from about 6 to about 200. Other nonionic surfactants are the condensation products of alkylene oxides with 2 moles of fatty acids (i.e. alkylene oxide diesters of fatty acids). These materials have the general formula $RCO(X)_nOOCR$ wherein R is a C10-30 alkyl group, X is - $OCH_2CH_2$-(i.e. derived from ethylene glycol or oxide) or $-OCH_2CHCH_3$-(i.e. derived from propylene glycol or oxide), and n is an integer from about 6 to about 100. Other nonionic surfactants are the condensation products of alkylene oxides with fatty alcohols (i.e. alkylene oxide ethers of fatty alcohols). These materials have the general formula $R(X)_nOR'$ wherein R is a C10-30 alkyl group, X is $-OCH_2CH_2$-(i.e. derived from ethylene glycol or oxide) or $-OCH_2CHCH_3-$ (i.e. derived from propylene glycol or oxide), and n is an integer from about 6 to about 100 and R' is H or a C10-30 alkyl group. Still other nonionic surfactants are the condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol]. These materials have the general formula $RCO(X)_nOR'$ wherein R and R' are C10-30 alkyl groups, X is $-OCH_2CH_2$ (i.e. derived from ethylene glycol or oxide) or $-OCH_2CHCH_3-$ (derived from propylene glycol or oxide), and n is an integer from about 6 to about 100. Nonlimiting examples of these alkylene oxide derived nonionic surfactants include ceteth-6, ceteth-10, ceteth-12, ceteareth-6, ceteareth-10, ceteareth-12, steareth-6, steareth-10, steareth-12, PEG-6 stearate, PEG-10 stearate, PEG-100 stearate, PEG-12 stearate, PEG-20 glyceryl stearate, PEG-80 glyceryl tallowate, PEG-10

glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Still other useful nonionic surfactants include polyhydroxy fatty acid amide surfactants corresponding to the structural formula:

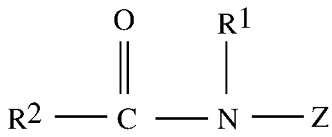

wherein: $R^1$ is H, $C_1$-$C_4$ alkyl, 2-hydroxyethyl, 2-hydroxy- propyl, preferably $C_1$-$C_4$ alkyl, more preferably methyl or ethyl, most preferably methyl; $R^2$ is $C_5$-$C_{31}$ alkyl or alkenyl, preferably $C_7$-$C_{19}$ alkyl or alkenyl, more preferably $C_9$-$C_{17}$ alkyl or alkenyl, most preferably $C_{11}$-$C_{15}$ alkyl or alkenyl; and Z is a polhydroxyhydrocarbyl moiety having a linear hydrocarbyl chain with a least 3 hydroxyls directly connected to the chain, or an alkoxylated derivative (preferably ethoxylated or propoxylated) thereof. Z preferably is a sugar moiety selected from the group consisting of glucose, fructose, maltose, lactose, galactose, mannose, xylose, and mixtures thereof. An especially preferred surfactant corresponding to the above structure is coconut alkyl N-methyl glucoside amide (i.e., wherein the $R^2CO$- moiety is derived from coconut oil fatty acids). Processes for making compositions containing polyhydroxy fatty acid amides are disclosed, for example, in G.B. Patent Specification 809,060, published February 18, 1959, by Thomas Hedley & Co., Ltd.; U.S. Patent No. 2,965,576, to E. R. Wilson, issued December 20, 1960; U.S. Patent No. 2,703,798, to A. M. Schwartz, issued March 8, 1955; and U.S. Patent No. 1,985,424, to Piggott, issued December 25, 1934.

Preferred among the nonionic surfactants are those selected from the group consisting of steareth-21, ceteareth-20, ceteareth-12, sucrose cocoate, steareth-100, PEG-100 stearate, and mixtures thereof.

Other nonionic surfactants suitable for use herein include sugar esters and polyesters, alkoxylated sugar esters and polyesters, C1-C30 fatty acid esters of C1-C30 fatty alcohols, alkoxylated derivatives of C1-C30 fatty acid esters of C1-C30 fatty alcohols, alkoxylated ethers of C1-C30 fatty alcohols, polyglyceryl esters of C1-C30 fatty acids, C1-C30 esters of polyols, C1-C30 ethers of polyols, alkyl phosphates, polyoxyalkylene fatty ether phosphates, fatty acid amides, acyl lactylates, and mixtures thereof. Nonlimiting examples of these non-silicon-containing emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, PPG-2 methyl glucose ether distearate, PEG-100 stearate, and mixtures thereof.

## Fatty Alcohols

Liquid foamable compositions herein include a fatty alcohol. For example, the liquid foamable composition can include monohydric saturated straight-chain fatty alcohols, such as one or more of behenyl alcohol, cetyl alcohol, and stearyl alcohol, and other waxy fatty alcohols having melting points of about 25°C or higher, or of about 45°C or higher; and at levels of about 10% or less, by weight of the liquid foamable composition; and about 4% or less, by weight of the liquid foamable composition.

In certain examples, the fatty alcohols can be non-volatile and have a low melting point. For example, such fatty alcohols can have a melting point of 30°C or less, about 25°C or less, or about 22°C or less. Unsaturated fatty alcohols can also be non-volatile. Suitable fatty alcohols can include unsaturated monohydric straight-chain fatty alcohols, saturated branched-chain fatty alcohols, saturated $C_8$-$C_{12}$ straight-chain fatty alcohols, and mixtures thereof. The unsaturated straight-chain fatty alcohols can typically have one degree of unsaturation. Di- and tri-unsaturated alkenyl chains can be present at low levels; about 5% or less, by total weight of the unsaturated straight-chain fatty alcohol; about 2% or less, by total weight of the unsaturated straight-chain fatty alcohol; and about 1% or less, by total weight of the unsaturated straight-chain fatty alcohol. The unsaturated straight-chain fatty alcohols can have an aliphatic chain size of from $C_{12}$-$C_{22}$ in certain examples, from $C_{12}$-$C_{18}$ in certain examples, and from $C_{16}$-$C_{18}$ in certain examples. Exemplary alcohols of this type can include oleyl alcohol and palmitoleic alcohol.

Branched-chain alcohols can typically have aliphatic chain sizes of from $C_{12}$-$C_{22}$, $C_{14}$-$C_{20}$ in certain examples, and $C_{16}$-$C_{18}$ in certain examples. Suitable branched-chain alcohols can include isostearyl alcohol, octyl dodecanol, and octyl decanol.

Examples of saturated $C_8$-$C_{12}$ straight-chain alcohols can include octyl alcohol, caprylic alcohol, decyl alcohol, and lauryl alcohol. Fatty alcohols having a low melting point can be included at levels from about 0.1% to about 10%, by weight of the liquid foamable composition, from about 0.2% to about 5%, by weight of the liquid foamable composition

in certain examples; and from about 0.5% to about 3%, by weight of the liquid foamable composition in certain examples.

It may be desirable to use waxy fatty alcohols for their conditioning benefits. However, if both waxy fatty alcohols and liquid fatty alcohols are present, a weight ratio of liquid to waxy fatty alcohols can be about 0.25 or less, in certain examples; about 0.15 or less, in certain examples; and about 0.10 or less, in certain examples.

A total amount of fatty alcohols in the liquid foamable composition can be from about 1% to about 10%, by weight; from about 2% to about 8%, by weight; and from about 3% to about 6%, by weight. In certain examples, a ratio of the fatty alcohol to the cationic surfactant can be about 2 parts to about 1 part. In such examples, the fatty alcohol and the cationic surfactant can combine to form liquid crystal structures in a lamellar gel phase. In examples where the ratio of the fatty alcohol to the cationic surfactant is lower (i.e., an amount of cationic surfactant is increased relative to an amount of fatty alcohol), the liquid crystal structures can be in the form of vesicles. In certain examples, the liquid crystal structures can be of any of a variety of suitable phases including, for example, bicontinuous cubic, hexagonal, inverse cubic, micellar cubic, reverse hexagonal columnar, and combinations thereof. Examples of liquid crystal structures are further described in U.S. Patent No. 8,470,305 and PCT International Publication No. WO 2010/060131.

Skin Care Actives

Skin care actives for use in the compositions herein are selected from niacinamide, hexyldecanol, a peptide, panthenol, undeylenoyl phenylalanine, retinyl propionate, and mixtures thereof. The compositions herein comprise from 12% to 25% , by weight, of a skin care active.

The liquid foamable composition can include water in amount such that water can provide a remainder of the liquid foamable composition. As such, a liquid foamable composition can include from about 20% to about 85%, by weight; from about 40% to about 85%, by weight; or from about 50% to about 85%, by weight, of water.

In certain examples, the water may include other liquid, water-miscible, or water-soluble solvents such as lower alkyl alcohols (e.g., $C_1$-$C_5$ alkyl monohydric alcohols), such as $C_2$-$C_3$ alkyl alcohols. However, the liquid fatty alcohol must be miscible in an aqueous portion of the liquid foamable composition. The fatty alcohol can be naturally miscible in the aqueous portion or can be made miscible through the use of co-solvents or surfactants.

The liquid foamable composition can also include a variety of other optional components suitable for rendering such compositions more cosmetically or aesthetically acceptable or to provide them with additional usage benefits. Such conventional optional ingredients can be well-known to those skilled in the art.

For example, the liquid foamable composition can also include one or more additional conditioning agents, such as those selected from the group consisting of avocado oil, fatty acids, hexyldecanol, isopropyl myristate, lanolin, apple wax, bees wax or jojoba oil, phospholipids (e.g., lecithines or ceramides), vaseline non-volatile hydrocarbons, and hydrocarbon esters. Imidazolidinyl derivatives, such as INCI Quaternium-87 (REWOQUAT® W 575 of Witco, Germany) can also be useful.

In certain examples, the liquid foamable composition can include a superabsorbent polymer. Suitable superabsorbent polymers can include polyacrylates (e.g., sodium polyacrylate starch) and polyacrylic acid polymers. Suitable materials are described, for example, in PCT Patent Applications WO 07/047598, WO 07/046052, WO2009/155265 and WO2009/155264. In certain examples, suitable superabsorbent polymer particles can be obtained by current state-of-the-art production processes, such as those described in WO 2006/083584. The superabsorbent polymers can be internally cross-linked (i.e., polymerization can be carried out in the presence of compounds having two or more polymerizable groups that can be free-radically copolymerized into the polymer network), externally surface crosslinked, or post crosslinked. Additional suitable superabsorbent polymers are described in U.S. Patent Publication No. 2013/0243836 and PCT International Application No. PCT/US2013/032922.

Preferably, the liquid foamable compositions are substantially free of thickening agents selected from carboxylic acid polymers, crosslinked polyacrylate polymers, polyacrylamide polymers, polysaccharides, and gums.

B. Propellant

A variety of conventional propellants (e.g., gases) can be used to transform the liquid foamable composition into a foam composition. Such propellants can include carbon dioxide and nitrous oxide. In certain examples, the propellant can be only one compound, and in other examples, the propellant can be a mixture of compounds. For example, in one example, only carbon dioxide can be used as a propellant. In certain examples, the propellant can include air. Other compounds can also be included to form the propellant in amounts of up to about 1%, by weight of the total propellant. These additional propellant compounds can include propane, butane, isobutane, dimethyl ether, and $N_2O$. These additional propellant compounds can be present without causing any disadvantages. In certain examples, the foam composition can include about 20 parts of propellant per one hundred parts.

In a bag-on-valve system, for example, a propellant can be held within a container, such that the propellant surrounds an inner bag. As described herein, propellants used in a bag-on-valve system can have minimal to no interaction

with a liquid foamable composition or a foam composition. As a result, types of propellant that can be used in a bag-on-valve system can be less restrictive than those used in examples where there is more interaction between the propellant and the liquid foamable composition or the foam composition. Suitable propellants for use in a bag-on-valve system can include, for example, hydrocarbons or any of a variety of suitable propellants.

In the foam composition, carbon dioxide can be included at levels of about 0.5% to about 20.0%, by weight, in certain examples; from about 1.0% to about 3.0%, by weight, in certain examples; and from about 1.5% to about 2.5%, by weight, in certain examples.

III. Aerosol Product

An aerosol product can include a liquid foamable composition, a propellant, and a package. In certain examples, the liquid foamable composition and propellant can be housed in the package, which can include a container and a valve, such that the liquid foamable composition and propellant can be combined and dispensed as a foam. In certain examples, a foam composition can be housed in a package.

The container can be any of a variety of aerosol containers or similar type containers known in the art. For example, the container can be a single chamber container or a barrier container. Non-limiting examples of single chamber containers can include plastic, glass, aluminum, or steel containers that can be unlined or lined with materials such as epoxy phenolics, organosols, and polyamide imides. In such single chamber containers, the liquid foamable composition and the propellant can be combined in the single chamber, as shown in FIG. 1. Barrier containers can keep the liquid foamable composition physically separate from the propellant within the container. Non-limiting examples of barrier containers can include a piston container and a bag-on-valve container, which are described in U.S. Patent Publication No. 2012/0288465.

The valve can be any of a variety of aerosol valves or similar type valves (e.g., any of a variety of valves supplied by APTAR®). In certain examples, the valve can be a powder valve. The powder valve can include one or more orifices on a valve stem, normally one or two orifices. Each of the orifices can have a same or different orifice diameter and can be in the form of any of a variety of shapes (e.g., circular, square, etc.). Both the orifice diameter and the orifice shape can be selected based upon the size and shape of the particulate material used in the liquid foamable composition. Further, certain valves, such as a powder valve, can help to prevent clogging of the aerosol product by wiping an opening of the orifice against a sealing gasket as the valve moves from an open position to a closed position. Non-limiting examples of suitable powder valve configurations are described in detail in U.S. Patent Nos. 3,773,064, 5,975,378, 6,394,321 and 8,580,725.

FIG. 1 shows a portion of a container 110 to which a valve is mounted, according to one example. A valve assembly 111 can generally include a dip tube 112, a valve housing 114, a valve-closing coil spring 116, and a valve body 118. The valve body 118 can have a hollow valve stem 120 extending upwardly therefrom and can include at least one orifice 122 leading into an interior of the valve stem 120. A sealing gasket 124, which can be made of rubber or other suitable resilient material, can surround the valve stem 120 and seal the orifice 122 when the valve is in the closed position. An actuator 126 having a nozzle 128 is shown to be attached to a top of the valve stem 120. When the actuator 126 is depressed downwardly against a force of the spring 116, the valve moves to the open position, and the orifice 122 can pass below the sealing gasket 124 such that the liquid foamable composition within the container can, under the influence of the propellant, pass up through the dip tube 112, into the valve body 118, through the orifice 122, into the valve stem 120, into the actuator 126, before being dispensed out through the nozzle 128. When the actuator 126 is released, the valve can return to the closed position, such that the spring 116 can push the valve stem 120 and the orifice 122 upwardly against the sealing gasket 124, wiping any remaining liquid foamable composition away from the orifice 122 of the valve stem 120 to prevent clogging of the orifice 122 and blocking flow of the liquid foamable composition.

The actuator 126 can be any of a variety of actuators known in the art. For example, an actuator can be a front-hinged, rear-hinged, or non-hinged actuator, as long as the actuator can be properly matched with the valve stem. Non-limiting examples of suitable hinged actuators can include those available from SEAQUIST® Perfect Dispensing under the trade names S30, S25, S20, and Allegra for upright containers and S16 and S4 for inverted containers. Non-hinged actuators can be used as they can tend to exhibit less lateral pressure during actuation of the aerosol product. Non-limiting examples of suitable non-hinged actuators can include those available from Precision Valve under the trade names City Spout, Hercules Spout, and Iris and those available from SEAQUIST® Perfect Dispensing under the trade name S2. Actuators, valves, containers, and other related parts and equipment can include those available from, for example, APTAR®, Precision Valve, and Summit Packaging Systems.

In another example, a container can include a bag-on-valve system, as mentioned herein and as shown in FIGS. 2 and 3. FIG. 2, for example, shows a bag-on-valve system including a container 210 having an inner bag 213, which can be filled with the foam composition or the liquid foamable composition, and an outer container 215, which can enclose the inner bag 213. A valve assembly 211, vertically movable between an open position and a closed position, can be attached to the inner bag 213.

The valve assembly 211 can include a housing 214, a valve stem 220, a spring 216, a valve plate 232, an inner sealing 234, and an outer sealing 236. The valve stem 220 can include one or more lateral openings 238. The spring 216 can be disposed between a lower end portion 240 of the valve stem 220 and the housing 214 and can bias the valve stem 220 upwardly towards the valve plate 232, which can be disposed at an upper end of the housing 214. The valve plate 232 can include two coaxially-arranged recesses 242, 244 extending in a circumferential direction of the valve plate 32. FIG. 2 shows an axial opening 246 located in a central portion of the inner recess 242. The inner sealing 234 can be disposed within the inner recess 242, attached to the valve plate 232, and can be adapted to engage the valve stem 220 such that the lateral opening 238 of the valve stem 220 is covered and blocked, respectively. The outer sealing 236 can be disposed in the second or outer recess 244 of the valve plate 232. The valve stem 220 can include a passage 248 in the central axial portion thereof, which can be connected to the lateral opening 238 on one side and connectable to a corresponding passage of a dispenser cap on the other side. In the closed position, a flow path from the interior space of the housing 214 along the valve stem 220 and through the lateral opening 238 can be blocked by the inner sealing 234.

The valve assembly 211 can be fixed to the inner bag 213 at an upper end thereof such that a lower end of the housing 214 of the valve assembly 211 can be gas-tight covered by the upper edge of the inner bag 213. Further, the inner bag 213 and the valve assembly 211 can be attached to the outer container 215 such that an upper end of the outer container 215 can engage the outer sealing 236 of the valve plate 232 in a gas-tight manner. Accordingly, an interior of the inner bag 213 and space between the outer container 215 and the inner bag 213 each can be independently sealed.

A dispenser cap having an actuator (not shown) can be attached to the valve plate 232 such that the actuator can engage the valve stem 220. When the actuator is depressed downwardly against a force of the spring 216, the valve assembly 211 can move to the open position. The valve stem 220 moves within the inner sealing 234, which can remain stationary, while contacting the same. Once the lateral opening 238 can be uncovered by the inner sealing 234, the flow path from the valve housing 214 through the lateral opening 238 can be opened. Thus, the interior of the inner bag 213 and the flow path inside the valve housing 214 become linked such that the foam composition/liquid foamable composition within the inner bag 213 can pass through the flow path and dispensed out of the dispenser cap by the pressure of the propellant/compressed gas, which can surround the inner bag 213.

As shown in FIG. 3, the inner bag 213 can include flat lateral edges 250 and a bottom fold 252, which can be directed towards an upper end of the inner bag 213 in order to allow a controlled collapse. Near the bottom fold 252, the inner bag 213 can include two flat triangular portions 254, each extending from the bottom edge 256 to the lateral edge 250 with an angle of about 45°. This can further facilitate the collapse of the inner bag 213, when compressed by the pressure of the propellant in the outer container 215 (as shown in FIG. 2). As described above, the outer container 215 can include any of a variety of propellants or any other suitable compressed gas. Pressure of the propellant can be set to from about 0.3 to about 1.0 MPa, or from about 0.3 to about 0.8 MPa, in order to stably discharge contents of the inner bag 213 as completely as possible.

The inner bag can be flexible, and can be made from any of a variety of suitable materials. In certain examples, the inner bag can be formed with a layer of a material that can be essentially impermeable to the propellant within the inner bag. In certain examples, the inner bag can be formed with a layer of a material that can be essentially impermeable to the propellant outside of the bag, as it may be required that such compositions do not mix during storage. Mixing of the propellant within the inner bag and the propellant outside of the bag can be inappropriate based on the properties of the foam composition/liquid foamable composition or any of a variety of other reasons. However, this does not preclude the possibility that the propellant within the inner bag and the propellant outside of the bag can be mixed upon dispensing of the foam composition/liquid foamable composition when a valve to dispense the foam is triggered. For example, a mixing channel (not shown) or another appropriate measure can be used in such a case to mix the respective propellants if desired.

IV. Method of Use

The foam composition can be used in conventional ways to improve sensory benefits to skin. This generally involves application of an effective amount of the foam composition to a portion of the skin of a user. For example, the foam composition can be dispensed from an aerosol can or similar container or package, and the foam composition can be applied and rubbed onto a desired portion of the skin of a user. An "effective amount" can refer to an amount sufficient enough to provide the desired sensory benefits, which can include, for example, a rich and creamy appearance and a favorable "feel."

In certain embodiments, the foam composition can provide the rich and creamy appearance and moisturization and protection capabilities associated with heavier products, while providing a rapid absorption and ease of application associated with lighter products. Furthermore, the foam composition can reduce or eliminate characteristics associated with a negative sensory experience such as, for example, tack, drag, and stickiness.

V. Test Methods

Bioactive Dose on substrate

The dose on counter test is a technical test used to quantify the amount of bioactive (niacinamide and glycerin) dosed per unit area when spreading a product on a black laminate substrate. The test doses 0.05 mL of product on a laminate substrate. The panelist then spreads the product covering as much surface area as possible in a rectangular shape until the product no longer spreads. Based upon the concentration of bioactives in the product, as well as the area covered during spread, the amount of bioactive per unit area can then be calculated.

Bioactive Dose on Face

The dose on face test is a technical test used to quantify the amount of bioactive dosed when spreading a product out on half of the face. Panelists are given a specified amount of product in a weigh boat. Panelists are then asked to dose as much product as required to cover half of their face (jaw to nose to forehead over half of the face). The weigh boat is then weighed again to determine how much product was used. The amount of bioactive dosed per half of the face can then be calculated using the amount of product dosed and the concentration of the bioactive in the product.

There is also a dose on face method that examines full face application with a larger number of panelists. Panelists are given a specified amount of product in a weigh boat, and are then asked to dose as much product as required to cover their entire face. The weigh boat is weighed after product application to determine how much product was used. The total amount of bioactive dosed is then calculated using the amount of product dosed and the concentration of the bioactive in the product.

Descriptive Analysis Panel (DAP)

The descriptive analysis panel (DAP) is a technical panel used to quantify rub in and aesthetic properties of products. DAP doses 0.15 mL of product to a circular area that is approximately 2 inches in diameter or 20 $cm^2$. DAP evaluates attributes such as rub out drag, cooling, shine, tack, and drag at multiple time points.

Specific Gravity (SG)

Specific gravity is measured using a pycnometer. First the mass of the empty pycnometer is measured. Next, the mass of the pycnometer plus water is measured, and the pycnometer is cleaned and dried. Finally, the mass of the pycnometer plus product is measured. Specific gravity is then calculated using the formula below.

$$\text{Specific Gravity} = \frac{(\text{Mass Product + Pycnometer} - \text{Mass Empty Pycnometer})}{(\text{Mass Water + Pycnometer} - \text{Mass Empty Pycnometer})}$$

Examples

Examples 1-5

The following representative examples illustrate compositions according to the present disclosure. The compositions in Examples 1- 5 are prepared by first combining the water phase ingredients in a container and mixing until uniform while heating to approximately 90°C. Fatty alcohols and surfactants are then added to the water phase and the temperature is then brought back up to 90°C while mixing. Compositions are then allowed to cool to approximately 40°C while continuing to mix. Once the batch reaches approximately 40°C, preservatives and retinoid package materials, if present, are added. Compositions continue to cool while mixing.

**Table 1**

| Ingredient | Example 1 Inventive | Example 2 Inventive | Example 3 Inventive | Example 4 Inventive | Example 5 Inventive |
|---|---|---|---|---|---|
| Water | QS | QS | QS | QS | QS |

(continued)

| | | | | | |
|---|---|---|---|---|---|
| Humectant | | | | | |
| Glycerin | 43 | 13 | 20 | 10 | 35 |
| Butylene Glycol | --- | 5 | --- | 2 | --- |
| Dipropylene Glycol | --- | 5 | --- | 2 | --- |
| Bioactives | | | | | |
| Niacinamide | 21 | 13 | 20 | 15 | 25 |
| dex-Panthenol | --- | --- | --- | 0.5 | --- |
| Sepiwhite[1] | --- | --- | --- | 1 | --- |
| Glyco-Repair[2] | --- | --- | --- | 2 | --- |
| Biomyox[3] | --- | --- | --- | 0.5 | --- |
| Palestrina[4] | --- | --- | --- | 0.3 | --- |
| Promatrixyl[5] | --- | --- | --- | 0.5 | --- |
| Retinoid Package | | | | | |
| Retinyl Propionate | --- | --- | 0.3 | --- | --- |
| Cyclopentasiloxane | --- | --- | 14 | --- | --- |
| Laureth-4 | --- | --- | 0.2 | --- | - |
| Fatty Alcohol | | | | | |
| Stearyl Alcohol | 2.4 | 9.8 | 4.9 | 1.2 | 4.9 |
| Cetyl Alcohol | 1 | 4 | 0.5 | 0.25 | 2 |
| Behenyl Alcohol | --- | --- | --- | 0.25 | --- |
| Batyl Alcohol | --- | --- | 1 | --- | --- |
| Nonionic Surfactant | | | | | |
| Emulgade PL 68/50[6] | --- | 2.9 | --- | 0.4 | --- |
| Stearic Acid | --- | 1.4 | --- | 0.2 | --- |
| PEG-100 Stearate | --- | 1.4 | --- | 0.2 | --- |
| Cationic Surfactant | | | | | |
| Behenyl Trimethyl Ammonium Chloride | 1.4 | --- | 2.9 | --- | --- |
| Behentrimonium Methosulfate | --- | --- | --- | --- | 2.9 |
| pH Adjustor | | | | | |
| Triethanolamine | --- | --- | --- | 0.45 | --- |
| Preservative | | | | | |
| Symdiol 68[7] | --- | --- | --- | 0.7 | --- |
| Phenoxyethanol | --- | --- | --- | 0.35 | --- |

(continued)

| | | | | | |
|---|---|---|---|---|---|
| Preservative | | | | | |
| Glydant Plus Liquid[8] | 0.3 | 0.3 | 0.3 | --- | 0.3 |

[1]Undecylenoyl Phenylalanine, from Seppic
[2]Water and hydrolyzed ceratonia silique seed extract, from Silab
[3]Water and nasturtrium officinale extract, from Silab
[4]Water, glycerin, decyl glucoside, lactic acid, benzyl alcohol, and palmitoyl dipeptide-7, from Sederma (France)
[5]Water, glycerin, PEG-100 stearate, benzyl alcohol, palmitoyl pentapeptide-3, from Sederma (France)
[6]Cetearyl Glucoside and Cetearyl Alcohol, from BASF
[7]1,2-hexanediol and caprylyl glycol, from Symrise
[8]DMDM Hydantoin, butane-1,3-diol, iodopropynyl butyl carbamate, water, from Lonza

Examples 6 - 12 - Impact of Foam on Bioactive Dose

Examples 6-12 demonstrate that foams deposited more bioactive per unit area compared to typical emsulsion products when normalizing the bioactive concentration dose based upon specific gravity.

The compositions of Examples 6 - 12 were created in the same manner as stated for Examples 1-5 above. Foams were created by weighing 200 grams of product into a stainless steel whip cream canister, and then adding two, 8 gram $CO_2$ cartridges and allowing the foam to gas overnight.

Table 2

| **Material** | **Example 6 - Water (% w/w) Comparative** | **Example 7 - Foam (% w/w) Inventive** |
|---|---|---|
| Water | 95.26 | 69.11 |
| Niacinamide | 2 | 13 |
| Glycerin | 2 | 13 |
| Behenyl Trimethyl Ammonium Chloride | 0.22 | 1.46 |
| Stearyl Alcohol | 0.37 | 2.44 |
| Cetyl Alcohol | 0.15 | 0.99 |
| $CO_2$ Propellant | - | 16 grams |
| Specific Gravity | 1 | 0.16 |

The dose on counter test was then conducted for examples 6 and 7 above. Data showed that Example 7 - Foam dosed 2.75 times more niacinamide and glycerin compared to Example 6 - Water. If spreading of the different forms was equivalent, the active dose would be the same between 6 and 7.

Example 6 - Water

0.05 mL * 1 SG * 20 mg Niacinamide = 1 mg Niacinamide
0.05 mL * 1 SG * 20 mg Glycerin = 1 mg Glycerin

Table 3

| **Panelist #** | **Area Dosed (cm)** | **Total Area ($cm^2$)** | **mg Niacinamide / $cm^2$** | **mg Glycerin / $cm^2$** |
|---|---|---|---|---|
| 1 | 6.5 x 3.5 | 22.75 | 0.04 | 0.04 |
| 2 | 7.5 x 3.5 | 26.25 | 0.04 | 0.04 |

(continued)

| Panelist # | Area Dosed (cm) | Total Area ($cm^2$) | mg Niacinamide / $cm^2$ | mg Glycerin / $cm^2$ |
|---|---|---|---|---|
| 3 | 7.5 x 3.0 | 22.50 | 0.04 | 0.04 |
| 4 | 7.0 x 5.0 | 35.00 | 0.03 | 0.03 |
| 5 | 12.0 x 3.0 | 36.00 | 0.03 | 0.03 |
| 6 | 8.0 x 3.5 | 28.00 | 0.04 | 0.04 |
| | | **Average** | **0.04** | **0.04** |

Example 7 - Foam

0.05 mL * 0.16 SG * 130 mg Niacinamide = 1 mg Niacinamide
0.05 mL * 0.16 SG * 130 mg Glycerin = 1 mg Glycerin

Table 4

| Panelist # | Area Dosed (cm) | Total Area ($cm^2$) | mg Niacinamide / $cm^2$ | mg Glycerin / $cm^2$ |
|---|---|---|---|---|
| 1 | 5.5 x 1.5 | 8.25 | 0.12 | 0.12 |
| 2 | 5.0 x 1.5 | 7.50 | 0.13 | 0.13 |
| 3 | 4.0 x 2.0 | 8.00 | 0.12 | 0.12 |
| 4 | 3.0 x 4.5 | 13.50 | 0.07 | 0.07 |
| 5 | 9.0 x 2.0 | 18.00 | 0.06 | 0.06 |
| 6 | 4.0 x 2.0 | 8.00 | 0.12 | 0.12 |
| | | **Average** | **0.11** | **0.11** |

The dose on face test was then conducted was then conducted for examples 6 and 7 above. Data showed that Example 7 - Foam dosed 2.8 times more niacinamide and glycerin compared to Example 6 - Water. This test allows panelists to use "as much as needed" and confirms that consumer usage doesn't reduce the dose under real life conditions.

Table 5

| | Example 6 - Water Comparative | | | Example 7 - Foam Inventive | | |
|---|---|---|---|---|---|---|
| **Panelist #** | **Product Dose** (g) | **Dose Nia/Gly (mg per ½ face)** | **Side of Face** | **Product Dose (g)** | **Dose Nia/Gly (mg per ½ face)** | **Side of Face** |
| 1 | 0.53 | 10.6 | Right | 0.11 | 14.3 | Left |
| 2 | 0.22 | 4.4 | Left | 0.16 | 20.8 | Right |
| 3 | 0.51 | 10.2 | Right | 0.19 | 24.7 | Left |
| 4 | 0.19 | 3.8 | Left | 0.11 | 14.3 | Right |
| 5 | 0.18 | 3.6 | Right | 0.12 | 15.6 | Left |
| 6 | 0.25 | 5.0 | Left | 0.13 | 16.9 | Right |
| | **Average** | **6.3** | | **Average** | **17.8** | |

Examples 8 and 9

Table 6

| Material | Example 8 - Water (% w/w) Comparative | Example 9 - Foam (% w/w) Inventive |
|---|---|---|
| Water | 83.07 | 29.47 |
| Niacinamide | 5.25 | 21.88 |
| Glycerin | 10.5 | 43.75 |
| Behenyl Trimethyl Ammonium Chloride | 0.35 | 1.46 |
| Stearyl Alcohol | 0.59 | 2.44 |
| Cetyl Alcohol | 0.25 | 1 |
| $CO_2$ Propellant | - | 16 grams |
| Specific Gravity | 1 | 0.24 |

The dose on counter test was then conducted for examples 8 and 9 above. Data showed that Example 9 - Foam dosed 3 times more niacinamide and 2.8 times more glycerin compared to Example 8 - Water.

Example 8 - Water

0.05 mL * 1 SG * 52.5 mg Niacinamide = 2.6 mg Niacinamide
0.05 mL * 1 SG * 105 mg Glycerin = 5.3 mg Glycerin

Table 7

| Panelist # | Area Dosed (cm) | Total Area ($cm^2$) | mg Niacinamide / $cm^2$ | mg Glycerin / $cm^2$ |
|---|---|---|---|---|
| 1 | 16.0 x 4.0 | 64.00 | 0.04 | 0.08 |
| 2 | 10.5 x 7.5 | 78.75 | 0.03 | 0.07 |
| 3 | 8.0 x 3.0 | 24.00 | 0.11 | 0.22 |
| 4 | 9.0 x 4.0 | 36.00 | 0.07 | 0.15 |
| 5 | 9.0 x 4.0 | 36.00 | 0.07 | 0.15 |
| | | **Average** | **0.06** | **0.13** |

Example 9 - Foam

0.05 mL * 0.16 SG * 219 mg Niacinamide = 2.6 mg Niacinamide
0.05 mL * 0.16 SG * 438 mg Glycerin = 5.3 mg Glycerin

Table 8

| Panelist # | Area Dosed (cm) | Total Area ($cm^2$) | mg Niacinamide / $cm^2$ | mg Glycerin / $cm^2$ |
|---|---|---|---|---|
| 1 | 5.5 x 2.5 | 13.75 | 0.19 | 0.39 |
| 2 | 7.0 x 2.5 | 17.50 | 0.15 | 0.30 |
| 3 | 4.0 x 2.5 | 10.00 | 0.26 | 0.53 |

(continued)

| Panelist # | Area Dosed (cm) | Total Area ($cm^2$) | mg Niacinamide / $cm^2$ | mg Glycerin / $cm^2$ |
|---|---|---|---|---|
| 4 | 5.5 x 4.0 | 22.00 | 0.12 | 0.24 |
| 5 | 5.0 x 3.0 | 15.00 | 0.17 | 0.35 |
| | | **Average** | **0.18** | **0.36** |

Examples 10 and 11

Table 9

| Material | Example 10 - Water (% w/w) Comparative | Example 11 - Foam (% w/w) Inventive |
|---|---|---|
| Water | 70.65 | 29.47 |
| Niacinamide | 9.10 | 21.88 |
| Glycerin | 18.20 | 43.75 |
| Behenyl Trimethyl Ammonium Chloride | 0.61 | 1.46 |
| Stearyl Alcohol | 1.02 | 2.44 |
| Cetyl Alcohol | 0.42 | 1 |
| $CO_2$ Propellant | --- | 16 grams |
| Specific Gravity | 1 | 0.24 |

The dose on counter test was then conducted for examples 10 and 11 above. Data showed that Example 11 - Foam dosed 2 times more niacinamide and 1.9 times more glycerin compared to Example 10 - Water.

Example 10 - Water

0.05 mL * 1 SG * 52.5 mg Niacinamide = 2.6 mg Niacinamide
0.05 mL * 1 SG * 105 mg Glycerin = 5.3 mg Glycerin

Table 10

| Panelist # | Area Dosed (cm) | Total Area ($cm^2$) | mg Niacinamide / $cm^2$ | mg Glycerin / $cm^2$ |
|---|---|---|---|---|
| 1 | 9.0 x 8.5 | 76.50 | 0.06 | 0.12 |
| 2 | 8.0 x 3.5 | 28.00 | 0.16 | 0.33 |
| 3 | 9.0 x 6.5 | 58.50 | 0.08 | 0.16 |
| 4 | 12.0 x 5.0 | 60.00 | 0.08 | 0.15 |
| 5 | 7.0 x 3.5 | 24.50 | 0.19 | 0.37 |
| | | **Average** | **0.11** | **0.23** |

Example 11 - Foam

0.05 mL * 0.16 SG * 219 mg Niacinamide = 2.6 mg Niacinamide
0.05 mL * 0.16 SG * 438 mg Glycerin = 5.3 mg Glycerin

Table 11

| Panelist # | Area Dosed (cm) | Total Area ($cm^2$) | mg Niacinamide / $cm^2$ | mg Glycerin / $cm^2$ |
|---|---|---|---|---|
| 1 | 4.5 x 7.5 | 33.75 | 0.14 | 0.27 |
| 2 | 6.0 x 3.0 | 18.00 | 0.26 | 0.51 |
| 3 | 8.0 x 5.0 | 40.00 | 0.12 | 0.23 |
| 4 | 8.0 x 3.0 | 24.00 | 0.19 | 0.38 |
| 5 | 5.0 x 2.5 | 12.50 | 0.37 | 0.74 |
| | | **Average** | **0.22** | **0.43** |

Example 12

Table 12

| Material | Example 12 - Foam Inventive |
|---|---|
| Water | 33.255 |
| Niacinamide | 21 |
| Glycerin | 43 |
| Behenyl Trimethyl Ammonium Chloride | 0.73 |
| Stearyl Alcohol | 0.495 |
| Cetyl Alcohol | 1.22 |
| $CO_2$ Propellant | 16 grams |
| Specific Gravity | 0.21 |

The large base, full face, dose on face test was conducted for Example 12 - Foam vs. Olay Total Effects™. Based upon panelists' usage data, Example 12 - Foam deposited 1.8 times more niacinamide and 2.6 times more glycerin.

Table 13

| Product | Niacinamide Dosed per Full Face (mg) | Glycerin Dosed per Full Face (mg) |
|---|---|---|
| **Example 12 - Foam Inventive** | 53 | 29 |
| **Olay Total Effects Comparative** | 108 | 41 |

Examples 13-15 - Sensorial Impact of Foams

Examples 13-15 utilize DAP testing to demonstrate the fast absorption and lack of cooling offered by foams vs. traditional water products. Examples 13-15 also demonstrate the importance foaming had on product aesthetics vs. using a product with high concentrations of skin care actives.

The compositions of Examples 13-15 below were created in the same manner as stated for Examples 1-5 above. The foam of Example 14 was created by weighing 200 grams of product into a stainless steel whip cream canister, and then adding two, 8 gram $CO_2$ cartridges and allowing the foam to gas overnight.

Table 14

| Material | Example 13 - Concentrate (% w/w) Comparative | Example 14 - Foam (% w/w) Inventive | Example 15 - Water (% w/w) Comparative |
|---|---|---|---|
| Water | 68.81 | 68.81 | 96.25 |
| Niacinamide | 13 | 13 | 1.56 |
| Glycerin | 13 | 13 | 1.56 |
| Behenyl Trimethyl Ammonium Chloride | 1.46 | 1.46 | 0.18 |
| Stearyl Alcohol | 2.44 | 2.44 | 0.29 |
| Cetyl Alcohol | 0.99 | 0.99 | 0.12 |
| Glydant Plus Liquid | 0.3 | 0.3 | 0.04 |
| $CO_2$ Propellant | - | 16 grams | - |
| Specific Gravity | 1 | 0.12 | 1 |

The DAP data detailed below show that Example 14 - Foam had lower cooling and shine, as well as faster rub in (rub out drag attribute) compared to both Example 13 - Concentrate and Example 15 - Water. The DAP data also showed that Example 13 - Concentrate had higher tack, residue, and drag compared to Example 14 - Foam and Example 15 - Water. Note that statistics were conducted using a Student's T-test with a 90% confidence interval.

Table 15

| DAP Attribute | Example 13 - Concentrate Comparative | Example 14 - Foam Inventive | Example 15 - Water Comparative |
|---|---|---|---|
| Cool Feel | 3.1c | 0.0d | 4.6b |
| Shine | 5.8a | 1.6d | 4.5b |
| Rub Out Drag | 1.6b | 3.1a | 0.5c |
| Tackiness | 3.2a | 1.9b | 1.2b |
| Amount of Residue | 5.2a | 1.8c | 2.6c |

## Claims

1. A liquid foamable composition, comprising:

   0.5% to 7%, preferably 0.5% to 5%, by weight, of a surfactant selected from cationic surfactants, nonionic surfactants, and mixtures thereof;
   1% to 15%, preferably 2% to 7%, by weight, of a fatty alcohol;
   12% to 25%, by weight, of a skin care active, wherein the skin care active is selected from niacinamide, hexyldecanol, a peptide, panthenol, undeylenoyl phenylalanine, retinyl propionate, and mixtures thereof; and
   water, wherein the composition comprises 5% to 30% by weight of a skin care active.

2. The composition of claim 1, further comprising 20% to 85%, preferably, 40% to 85%, and more preferably, 50% to 85%, by weight, of water.

3. The composition of claim 1 or 2, further comprising 5% to 50%, by weight, of a polyol humectant, wherein the polyol humectant preferably contains at least 50% glycerin, and more preferably the polyol humectant contains at least 75% glycerin.

4. The composition of any preceding claim, wherein the surfactant is nonionic and is selected from cetearyl glucoside,

stearic acid, PEG-100 stearate, glyceryl monostearate, and mixtures thereof.

**5.** The composition of any preceding claim, further comprising a propellant selected from hydrocarbons (A46, A70), hydrofluoro-olefins, carbon dioxide, and mixtures thereof.

**6.** The composition of claim 5, wherein the propellant is a hydrocarbon present at a concentration of 0.5% to 10%, preferably 1% to 5%.

**7.** The composition of claim 5, wherein the propellant is a hydrofluoro-olefin present at a concentration of 0.5% to 35%, preferably 5% to 25%.

**8.** The composition of claim 5, wherein the propellant is carbon dioxide present at a concentration of 0.5% to 20%, preferably 2% to 10%, and more preferably 4% to 8%.

**9.** The composition of any preceding claim, wherein the liquid foamable composition is substantially free of a thickening agent selected from carboxylic acid polymers, crosslinked polyacrylate polymers, polyacrylamide polymers, polysaccharides and gums.

**10.** The composition of any preceding claim, wherein the fatty alcohol has a melting point of 25°C or higher, and preferably the fatty alcohol is selected from behenyl alcohol, cetyl alcohol, batyl alcohol, stearyl alcohol, and mixtures thereof.

**11.** The composition of any preceding claim, wherein the surfactant is a quaternary ammonium compound selected from the group consisting of behenyl trimethyl ammonium chloride, stearamidopropyl dimethylamine, behentrimonium methosulfate, behenylamidopropyl dimethylamine, stearyl ethylhexyldimonium methosulfate, dicetyldimonium chloride, ditallow dimethyl ammonium chloride, and mixtures thereof.

**12.** The composition of claim 5, 6, 7, or 8, wherein the foam has a specific gravity measured with a pycnometer as specified herein of less than 0.6, preferably, less than 0.4 and even more preferably, less than 0.3.

**Patentansprüche**

**1.** Flüssige, schäumbare Zusammensetzung, umfassend:

zu 0,5 Gew.-% bis 7 Gew.-%, vorzugsweise zu 0,5 Gew.-% bis 5 Gew.-% ein Tensid, ausgewählt aus kationischen Tensiden, nichtionischen Tensiden und Mischungen davon;
zu 1 Gew.-% bis 15 Gew.-%, vorzugsweise zu 2 Gew.-% bis 7 Gew.-% einen Fettalkohol;
zu 12 Gew.-% bis 25 Gew.-% einen Hautpflegewirkstoff, wobei der Hautpflegewirkstoff ausgewählt ist aus Niacinamid, Hexyldecanol, einem Peptid, Panthenol, Undeylenoylphenylalanin, Retinylpropionat und Mischungen davon; und
Wasser, wobei die Zusammensetzung zu 5 Gew.-% bis 30 Gew.-% einen Hautpflegewirkstoff umfasst.

**2.** Zusammensetzung nach Anspruch 1, ferner umfassend zu 20 Gew.-% bis 85 Gew.-%, vorzugsweise zu 40 Gew.-% bis 85 Gew.-% und bevorzugter zu 50 Gew.-% bis 85 Gew.-% Wasser.

**3.** Zusammensetzung nach Anspruch 1 oder 2, ferner umfassend zu 5 Gew.-% bis 50 Gew.-% ein Polyol-Feuchthaltemittel, wobei das Polyol-Feuchthaltemittel vorzugsweise zu mindestens 50 % Glycerin enthält, und wobei stärker bevorzugt das Polyol-Feuchthaltemittel zu mindestens 75 % Glycerin enthält.

**4.** Zusammensetzung nach einem der vorstehenden Ansprüche, wobei das Tensid nichtionisch ist und ausgewählt ist aus Cetearylglucosid, Stearinsäure, PEG-100-Stearat, Glycerylmonostearat und Mischungen davon.

**5.** Zusammensetzung nach einem der vorstehenden Ansprüche, ferner umfassend ein Treibmittel, ausgewählt aus Kohlenwasserstoffen (A46, A70), Hydrofluorolefinen, Kohlendioxid und Mischungen davon.

**6.** Zusammensetzung nach Anspruch 5, wobei das Treibmittel ein Kohlenwasserstoff ist, der in einer Konzentration von 0,5 % bis 10 %, vorzugsweise 1 % bis 5 %, vorliegt.

7. Zusammensetzung nach Anspruch 5, wobei das Treibmittel ein Hydrofluorolefin ist, das in einer Konzentration von 0,5 % bis 35 %, vorzugsweise 5 % bis 25 %, vorliegt.

8. Zusammensetzung nach Anspruch 5, wobei das Treibmittel Kohlendioxid in einer Konzentration von 0,5 % bis 20 %, vorzugsweise von 2 % bis 10 %, und mehr bevorzugt von 4 % bis 8 %, vorliegt.

9. Zusammensetzung nach einem der vorstehenden Ansprüche, wobei die flüssige, schäumbare Zusammensetzung im Wesentlichen frei von einem Verdickungsmittel ist, ausgewählt aus Carbonsäurepolymeren, vernetzten Polyacrylatpolymeren, Polyacrylamidpolymeren, Polysacchariden und Gummistoffen.

10. Zusammensetzung nach einem der vorstehenden Ansprüche, wobei der Fettalkohol einen Schmelzpunkt von 25 °C oder höher aufweist, und vorzugsweise der Fettalkohol ausgewählt ist aus Behenylalkohol, Cetylalkohol, Batylalkohol, Stearylalkohol und Mischungen davon.

11. Zusammensetzung nach einem der vorstehenden Ansprüche, wobei das Tensid eine quartäre Ammoniumverbindung ist, die ausgewählt ist aus der Gruppe bestehend aus Behenyltrimethylammoniumchlorid, Stearamidpropyldimethylamin, Behentrimoniummethosulfat, Behenylamidpropyldimethylamin, Stearylethylhexyldimoniummethosulfat, Dicetyldimoniumchlorid, Di-talgdimethylammoniumchlorid und Mischungen davon.

12. Zusammensetzung nach Anspruch 5, 6, 7 oder 8, wobei der Schaum eine spezifische Schwerkraft aufweist, die mit einem Pyknometer wie hierin angegeben von weniger als 0,6, vorzugsweise weniger als 0,4 und noch bevorzugter weniger als 0,3 gemessen wird.

## Revendications

1. Composition moussable liquide, comprenant :

   0,5 % à 7 %, de préférence 0,5 % à 5 %, en poids, d'un agent tensioactif choisi parmi des agents tensioactifs cationiques, des agents tensioactifs non ioniques, et des mélanges de ceux-ci ;
   1 % à 15 %, de préférence 2 % à 7 %, en poids, d'un alcool gras ;
   12 % à 25 %, en poids, d'un principe actif de soin de la peau, dans laquelle le principe actif de soin de la peau est choisi parmi le niacinamide, l'hexyldécanol, un peptide, le panthénol, l'undécylénoyl-phénylalanine, le propionate de rétinyle, et des mélanges de ceux-ci ; et
   de l'eau, dans laquelle la composition comprend 5 % à 30 % en poids d'un principe actif de soin de la peau.

2. Composition selon la revendication 1, comprenant en outre 20 % à 85 %, de préférence, 40 % à 85 %, et plus préférablement, 50 % à 85 %, en poids, d'eau.

3. Composition selon la revendication 1 ou 2, comprenant en outre 5 % à 50 %, en poids, d'un humectant polyol, dans laquelle l'humectant polyol contient de préférence au moins 50 % de glycérine, et plus préférablement l'humectant polyol contient au moins 75 % de glycérine.

4. Composition selon l'une quelconque revendication précédente, dans laquelle l'agent tensioactif est non ionique et est choisi parmi le glucoside cétéarylique, l'acide stéarique, le stéarate de PEG-100, le monostéarate de glycéryle, et des mélanges de ceux-ci.

5. Composition selon l'une quelconque revendication précédente, comprenant en outre un propulseur choisi parmi des hydrocarbures (A46, A70), des hydrofluorooléfines, le dioxyde de carbone, et des mélanges de ceux-ci.

6. Composition selon la revendication 5, dans laquelle le propulseur est un hydrocarbure présent en une concentration de 0,5 % à 10 %, de préférence de 1 % à 5 %.

7. Composition selon la revendication 5, dans laquelle le propulseur est une hydrofluorooléfine présente en une concentration de 0,5 % à 35 %, de préférence de 5 % à 25 %.

8. Composition selon la revendication 5, dans laquelle le propulseur est le dioxyde de carbone présent en une concentration de 0,5 % à 20 %, de préférence de 2 % à 10 %, et plus préférablement de 4 % à 8 %.

9. Composition selon l'une quelconque revendication précédente, dans laquelle la composition moussable liquide est essentiellement dépourvue d'un agent épaississant choisi parmi des polymères d'acides carboxyliques, des polymères polyacrylates réticulés, des polymères polyacrylamides, des polysaccharides et des gommes.

10. Composition selon l'une quelconque revendication précédente, dans laquelle l'alcool gras a un point de fusion supérieur ou égal à 25 °C, et de préférence l'alcool gras est choisi parmi l'alcool béhénylique, l'alcool cétylique, l'alcool batylique, l'alcool stéarylique, et des mélanges de ceux-ci.

11. Composition selon l'une quelconque revendication précédente, dans laquelle l'agent tensioactif est un composé d'ammonium quaternaire choisi dans le groupe constitué par le chlorure de béhényltriméthylammonium, la stéaramidopropyldiméthylamine, le méthosulfate de béhentrimonium, la béhénylamidopropyldiméthylamine, le méthosulfate de stéaryléthylhexyldimonium, le chlorure de dicétyldimonium, le chlorure de disuifdiméthylammonium, et des mélanges de ceux-ci.

12. Composition selon les revendications 5, 6, 7, ou 8, dans laquelle la mousse a une gravité spécifique mesurée avec un pycnomètre tel que spécifié ici inférieure à 0,6, de préférence, inférieure à 0,4 et encore plus préférablement, inférieure à 0,3.

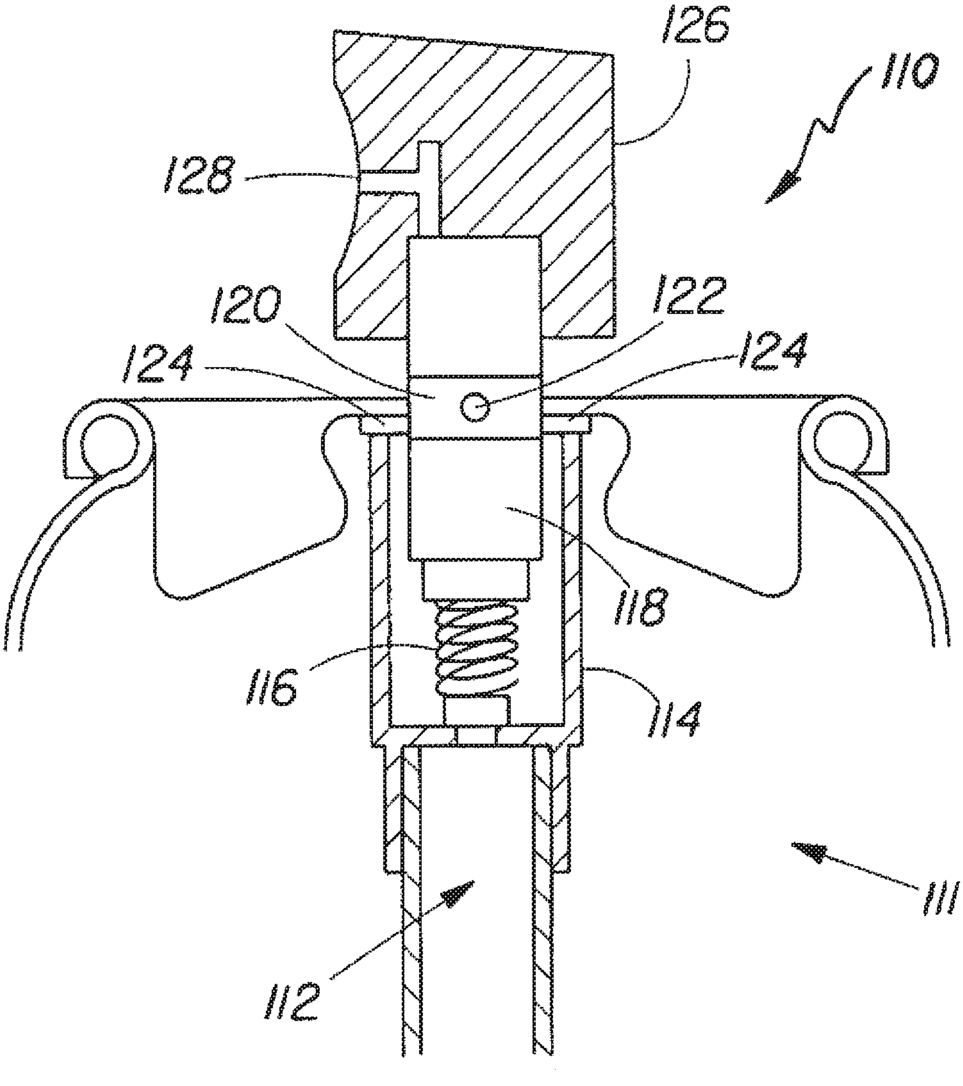


FIG. 1

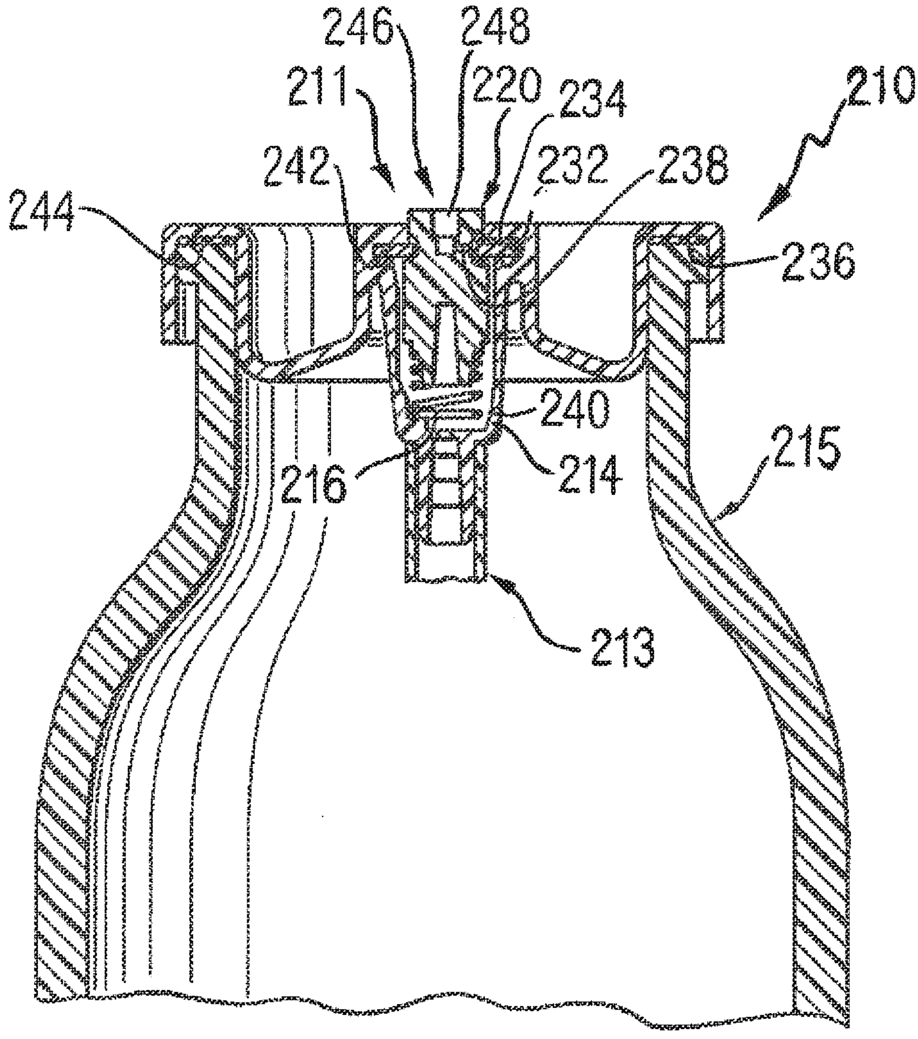


FIG. 2

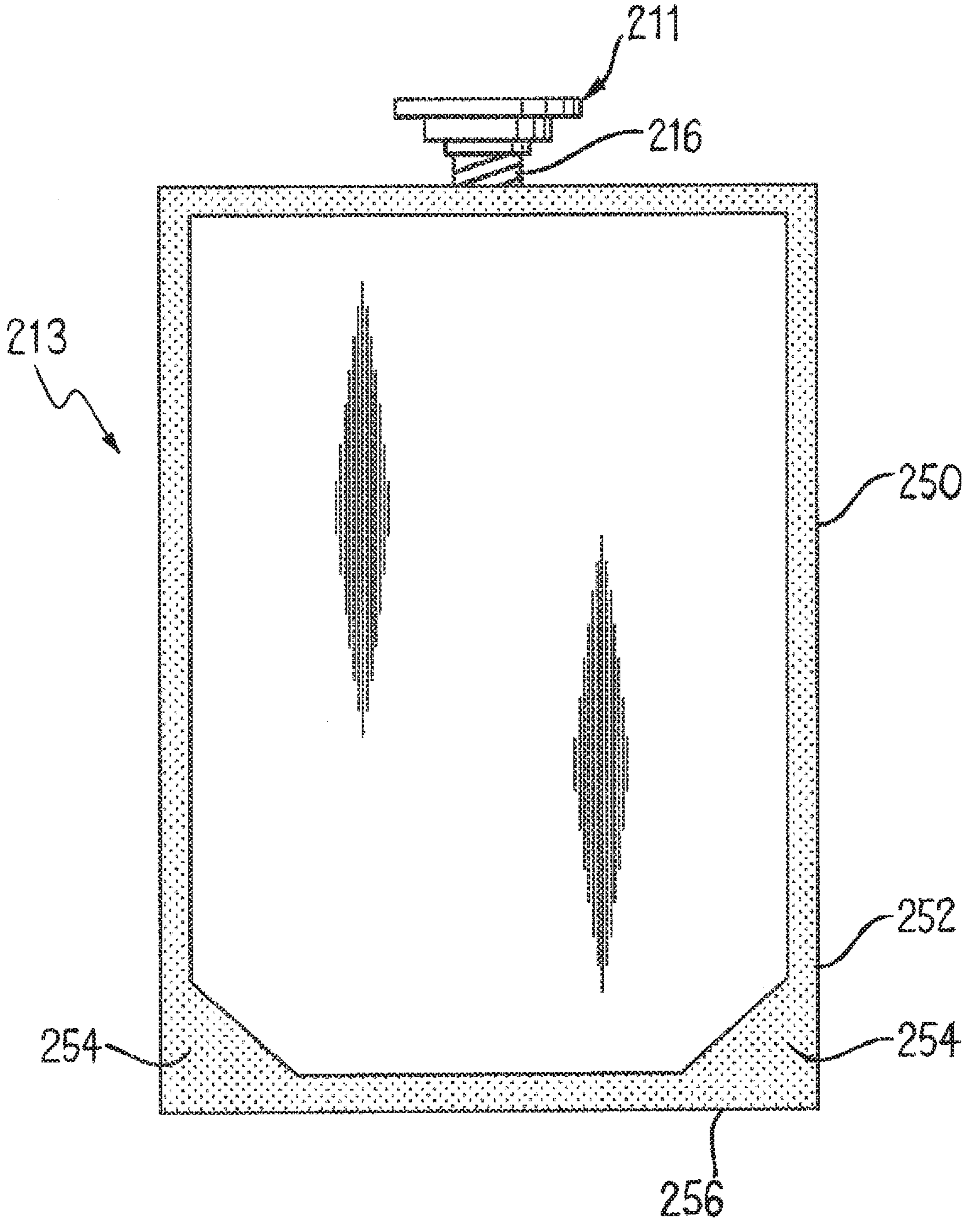


FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2004191205 A
- US 3155591 A, Hilfer
- US 3929678 A, Laughlin
- US 3959461 A, Bailey
- US 4387090 A, Bolich, Jr.
- US 8936798 B
- US 4275055 A, Nachtigal
- GB 809060 A
- US 2965576 A, E. R. Wilson
- US 2703798 A, A. M. Schwartz
- US 1985424 A, Piggott
- US 8470305 B
- WO 2010060131 A
- WO 07047598 A
- WO 07046052 A
- WO 2009155265 A
- WO 2009155264 A
- WO 2006083584 A
- US 20130243836 A
- US 2013032922 W
- US 20120288465 A
- US 3773064 A
- US 5975378 A
- US 6394321 B
- US 8580725 B


### Non-patent literature cited in the description


- McCutcheon's, Detergents & Emulsifiers. 1979
- **SCHWARTZ et al.** Surface Active Agents, Their Chemistry and Technology, New York. Interscience Publishers, 1949